(19) Europäisches Patentamt
European Patent Office
Office européen des brevets

(11) Publication number: **0 030 969**
**B1**

(12) **EUROPEAN PATENT SPECIFICATION**

(45) Date of publication of patent specification: **03.10.84**

(21) Application number: **80901348.5**

(22) Date of filing: **22.05.80**

(88) International application number: **PCT/US80/00612**

(87) International publication number: **WO 81/00011 08.01.81 Gazette 81/01**

(51) Int. Cl.$^3$: **C 07 D 233/72**, C 07 D 233/96, C 07 D 239/22, C 07 D 239/55, C 07 D 239/62, C 07 D 251/32, B 32 B 15/08, B 32 B 17/10, B 32 B 27/06, B 32 B 27/08, B 32 B 27/16

# (54) POLY(ETHYLENICALLY UNSATURATED ALKOXY)HETEROCYCLIC COMPOUNDS AND CROSSLINKED POLYMERIC COATINGS.

(30) Priority: **25.06.79 US 51876**
**25.06.79 US 51877**

(43) Date of publication of application: **01.07.81 Bulletin 81/26**

(45) Publication of the grant of the patent: **03.10.84 Bulletin 84/40**

(84) Designated Contracting States: **DE FR GB NL**

(56) References cited:
SU-A- 477 164
US-A-3 562 275
US-A-3 808 226
US-A-3 821 098
US-A-3 847 769
US-A-3 852 302
US-A-3 894 016
US-A-3 914 165
US-A-3 968 305
US-A-4 014 771
US-A-4 024 146

(73) Proprietor: **MINNESOTA MINING AND MANUFACTURING COMPANY**
**3M Center, P.O. Box 33427**
**St. Paul, MN 55133 (US)**

(72) Inventor: **WENDLING, Larry A.**
**3M Center, P.O. Box 33427**
**St. Paul, MN 55133 (US)**

(74) Representative: **Bowman, Paul Alan et al**
**LLOYD WISE, TREGEAR & CO. Norman House**
**105-109 Strand**
**London WC2R OAE (GB)**

(56) References cited:
US-A-4 071 477
US-A-4 137 139
US-A-4 150 234
US-A-4 160 178

Journal of Applied Polymer Science, Vol. 15, issued April, 1971 (Easton, PA, USA), pp. 937-47, H. Alaminov et al, "Study of oligoester maleinates containing an isocyanuric ring by infrared spectroscopy"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 030 969 B1

(58) References cited:
Industrial & Engineering Chemistry, Product Research Development, Vol. 9, No. 3, issued 1970 (Easton, PA, USA), pp. 301-4, H. Alaminov et al, "Allylisocyanurates - radical polymerization with vinyl monomers"

Chemical Abstracts, Vol. 77, issued 10 July 1972 (Columbus, Ohio, USA), Abstract No. 6236t, K.H. Alaminov et al, "Thermooxidative decomposition of crosslinked copolymers of methyl methacrylate with allylic isocyanurates", Eur. Polym. J., 1972, 8(3), 361-8

Chemical Abstracts, Vol. 77, issued 30 October 1972 (Columbus, Ohio, USA), Abstract No. 115205c, K. Alaminov et al, "Derivatographic research on three-dimensional copolymers of methyl methacrylate and tris-N,N',N"-(2-hydroxy-3-allyloxypropyl)isocyanurate", Khim. Ind. (Sofia) 1972, 44(3), 102-3 (Bulg.)

## Description

### Technical Field

The present invention relates to ethylenically unsaturated crosslinking agents and to radiation curable compositions containing these agents.

### Background Art

The generation of three-dimensional bonding or crosslinking in a composition or coating to reduce the solubility and improve the chemical resistance of a cured product is well known. This is usually effected by the addition of a crosslinking agent to an otherwise two dimensionally polymerizable composition from which the cured product is made. Crosslinking has been produced in products from ethylenically unsaturated compositions such as acrylic compositions (e.g. a methyl methacrylate composition) by incorporation of from about 1 to about 10 percent by weight of a polyacrylic substituted compound as a crosslinking agent. It is well known that such acrylic compositions generally must be polymerized in an inert atmosphere, e.g. a nitrogen atmosphere. Otherwise, the oxygen present in air will retard or even prevent polymerization of the acrylic composition so that desired levels of polymerization cannot be achieved. At best, only a tacky, incompletely polymerized resin or a weak, low molecular weight polyacrylate resin can be obtained.

Curable, oxygen insensitive acrylic compositions are described in United States Patent Specification Nos. 3 844 916, 3 914 165 and 3 925 349. These references teach that oxygen inhibition can be avoided by incorporation of a Michael adduct of a polyacrylate and an amine having at least one amino hydrogen into acrylic compositions. The use of such an adduct in acrylic photopolymerizable compositions requires the use of a relatively high concentration of polymerization photoinitiator (3% by weight is disclosed at Col. 3, lines 50—51 of U.S. Patent 3,925,349). Although such compositions are useful for coatings and inks that can be cured in the presence of oxygen, these compositions are not satisfactory for coatings that are transparent and where discoloration is undesirable since the use of large amounts of photoinitiator leads to yellowing of the cured coating.

Acrylic compositions, containing 0.5 to 10 percent triphenyl phosphine, that can be cured rapidly in an atmosphere containing 300 to 1000 ppm of oxygen are disclosed in U.S. Patent 4,113,983. Since the provision of atmosphere containing oxygen in any concentration less than that found in air requires use of special equipment, the use of phosphines to obtain rapid curing is also unsatisfactory for many commercial processes.

U.S. Patent 3,968,305 describes acrylic compositions comprising an aliphatic compound having three or more methacryloxy groups that can be polymerized to a crosslinked mar resistant coating. U.S. Patent 4,014,771 teaches that by the addition of 1) 30 to 95 percent of the adduct of methacrylic acid and 2) either a polyglycidyl ether of an aromatic polyhydric compound or a polyglycidyl ester of an aromatic or aliphatic polycarboxylic acid to a polymethacryloxy compound such as that described in U.S. Patent 3,968,305, there is obtained a composition which evidently can be polymerized without the necessity of excluding air during the polymerization.

Protective coatings produced by irradiation in the absence of air of the adduct of methacrylic acid to N-glycidylheterocyclic compounds are disclosed in U.S. Patents 3,808,226 and 3,847,769. Polymerization of the dimethacrylic ester of N-oxyalkylated-heterocyclic compounds is disclosed in United States Patent Specifications Nos. 3 821 098 and 3 852 302.

The compounds of United States Patent Specification No. 3 808 226 bear a similarity in structure to the compounds of the present invention. The route of synthesis shown for those compounds can not produce the compounds of the present invention nor could the route of synthesis used in the present invention produce the compounds of that Patent.

Other compounds including a heterocyclic nucleus having unsaturated pendant groups are disclosed in United States Patent Specification Nos. 3 808 226, 3 894 016, 3 847 769 and 4 137 139 and Chem. Abs. *77*, 1972 Abstract 6236t and Chem. Abs. *77*, 1972, Abstract 115205c. These compounds, in common with others in the prior art, do not include an ether linkage between the terminal unsaturated portion of the pendant groups and the heterocyclic nucleus.

Russian Patent Specification No. 477 164, J. Applied Polymer Science *15*, 1971, pages 937 to 947, and Ind. and Eng. Chemistry, Prod. Res. Development, 9 (3), 1970 pages 301 to 304 disclose compounds including a heterocyclic nucleus having terminally unsaturated pendant groups incorporating an ether linkage. There is no disclosure of such compounds including acryloyl or methacryloyl as the terminal unsatured group and such compounds could not be prepared by the processes disclosed since the acryloyl and methacryloyl groups would polymerize in the presence of the catalysts used in the preparation.

### Disclosure of the Invention

According to the present invention there are provided compounds of the general formula:

$$A^1—Z—A^2 \qquad I$$

in which $A^1$ and $A^2$ independently are groups having terminal ethylenic unsaturation and having the general formula:

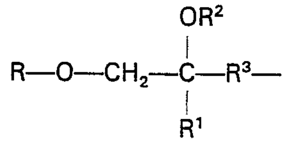

in which R—O— is a monovalent residue (formed by removal of the active hydrogen from an —OH group) of an aliphatic terminally unsaturated primary alcohol, ROH, R having the formula:

$$[E\!-\!(CH_2)_b\!-]_m R^5\!-\!(CH_2)_c \qquad II$$

in which:

E is 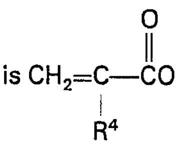

b is zero or an integer of 1 to 6,

c is an integer of 1 to 6,

$R^1$ and $R^4$ are independently hydrogen or methyl,

$R^5$ is an aliphatic group having 1 to 15 carbon atoms (preferably alkyl having m+1 hydrogens removed, e.g. alkylene where m=1, most preferably of up to 8 carbon atoms) which may be interrupted with up to two groups selected from ether oxygen atoms and

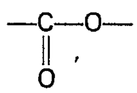 ,

and has a valence of m + 1, and

m is an integer of 1 to 5,

$R^2$ is preferably hydrogen but can be

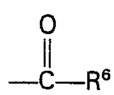 or 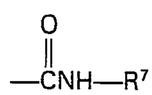

wherein $R^6$ is preferably alkenyl but can be alkyl (each preferably having 2 to 5 carbon atoms) and can be substituted by a phenyl or carboxyl group and $R^7$ is an aliphatic group (preferably of up to eight carbon atoms, e.g. alkyl) or aromatic group (preferably having up to 8 carbon atoms and more preferably a phenyl group) and $R^7$ is most preferably an acryloyloxyalkyl or a methacryloyloxyalkyl group,

$R^3$ is an alkylene group having 1 to 6 carbon atoms and up to one —O— radical in the group, and

Z is a heterocyclic group of the formula:

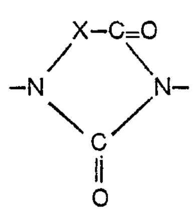

in which:

X is a divalent group which is required to complete a 5- or 6-membered heterocyclic ring, preferably X is

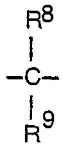 but X can be 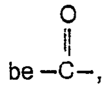, 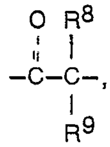, 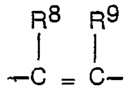, 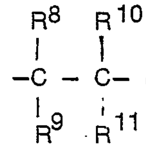 or 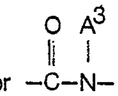

in which $R^8$, $R^9$, $R^{10}$ and $R^{11}$ are independently hydrogen or alkyl of 1 to 12 carbon atoms (preferably of 1 to 4 carbon atoms), cycloalkyl (of 3 to 6 carbon atoms) or an optionally substituted phenyl group of 6 to 12 carbon atoms, and $A^3$ is as defined above for $A^1$ and $A^2$.

The preferred compounds of formula I are those in which E is

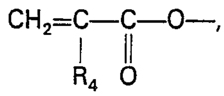

m is 2 to 5, and X is

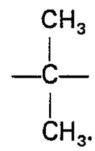

These compounds are preferred because they provide not only a high crosslink density, resulting in improved solvent abrasion resistance but also excellent adhesion and flexibility. Furthermore, these compounds are water/alcohol soluble and are photocurable to tack free surfaces in the presence of atmosphere oxygen.

Detailed Description of the Invention

The compounds of the invention can be prepared by the Lewis acid catalyzed addition of n moles of an ethylenically unsaturated primary alcohol to an epoxy-substituted heterocycle in accordance with the equation:

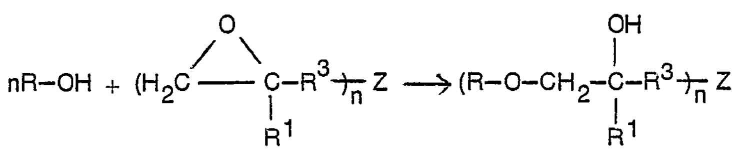

II

wherein R, $R^1$, $R^3$, and Z are as defined for the compounds of Formula I, and n is 2 or 3.

Particularly, the (polyacryloyloxy)alkoxypropylheterocyclic compounds of the invention are 5- or 6-membered ring heterocyclic compounds having preferably two (but may have three) nitrogen and preferably two (but may have three) carbonyl groups, viz.

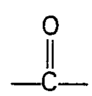

in the ring. At least one but preferably all of the ring nitrogens are substituted by a (polyacryloyloxy)-alkoxypropyl group (e.g., Formula II). The substituted heterocyclic compounds can be prepared (as shown above) by the Lewis acid catalyzed addition to a heterocyclic compound, as defined, that has one, two or three (where present) of its ring nitrogens substituted by a glycidyl group (e.g., a 2,3-epoxypropyl group), of one, two or three equivalents of a hydroxy compound that is the product of esterification of m hydroxyl groups of a polyol having (m + 1) hydroxyl groups with acrylic or methacrylic acid in accordance with the equation:

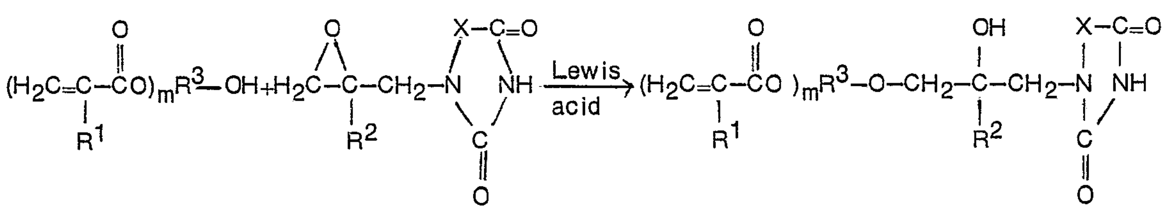


(IV)

wherein $R^1$, $R^2$, m, $R^3$ and X are defined above.

The above equation illustrates the preparation where only one of the ring nitrogens has been substituted by the glycidyl group. Where two or three of the ring nitrogens have been substituted by glycidyl (as is most preferable), two or three equivalents of hydroxy compound can be added. The addition of the hydroxy compound to the glycidyl groups of the heterocyclic compound can be done in

one step or in a sequence of steps in which first one and then a second and then a third glycidyl group is reacted. It is not necessary that the same hydroxyl compound be used in each of the steps. Where two or more different hydroxyl compounds are used, unsymmetrical compounds are obtained, that is, $A^1$ and $A^2$ (and $A^3$ if three nitrogens on the ring) of Formula I are different. Mixtures of hydroxyl compounds can also be used. It is to be expected, however, when two or more hydroxyl compounds are used, whether in a sequence of steps or in a one-step mixture, the product obtained will be a mixture of (polyacryloyloxy)alkoxypropylheterocyclic compounds. All, however, are useful in the present invention, particularly when at least about 30% by weight of the polymerizable coating composition is a heterocyclic compound having at least two glycidyl groups reacted with hydroxy compounds in which m in Formula I is at least three; that is, the hydroxy compound to be reacted with the glycidyl group of the heterocyclic compound is preferably a tri- or higher acryloyloxy or methacryloyloxy-hydroxy compound.

The polyglycidyl heterocyclic intermediates useful in the preparation of any and all of the compounds of the present invention are disclosed in U.S. Patents Nos. 3,808,226 and 4,071,477. Preferably, the reaction is performed in solution. However, it also can be performed in the absence of solvent. Generally, a solution of an epoxy-substituted heterocyclic can be added incrementally (over a period of time ranging from a few minutes to several hours) to a mixture of 1) an ethylenically unsaturated primary alcohol (or mixtures of ethylenically unsaturated primary alcohols), 2) an inhibitor for thermal polymerization, and 3) a Lewis acid while maintaining the temperature of the mixture at 50 to 120°C, preferably about 80 to 100°C, until the disappearance of the epoxy group, as indicated by chemical titration or nuclear magnetic resonance spectrometric analysis. Heating the mixture for from 2 to 40 hours usually suffices to complete the reaction, after which volatiles are removed by vacuum distillation.

The compounds of Formula II can then be acylated by reaction with an acylating agent, preferably an acyl halide, an acyl anhydride, or an isocyanate that contains polymerizable ethylenically unsaturated groups. Preferred acylated compounds have the Formula:

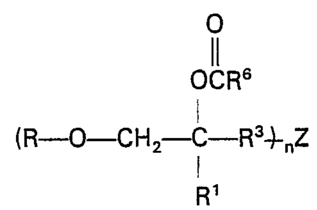

III

or

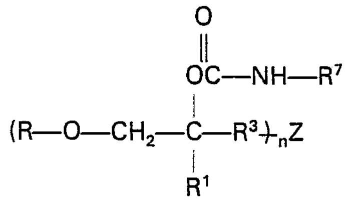

IV

wherein R, $R^1$, $R^3$, $R^6$, $R^7$, z, m and n are as defined for Formula I.

Exemplary acylating agents include acid chlorides such as acetyl chloride, propionyl chloride, valeryl chloride, dodecanyl chloride, acrylolyl chloride, methacryloyl chloride, alpha-chloroacryloyl chloride, croyl chloride, benzoyl chloride, phenylacetyl chloride, 2,4-dichlorophenylacetyl chloride; and the corresponding carboxylic acids and anhydrides; other anhydrides include the anhydrides of dicarboxylic acids such as maleic anhydride, succinic anhydride, methylenesuccinic anhydride, phthalic anhydride, and 3-chlorophthalic anhydride; and organic isocyanates such as methyl isocyanate, ethyl isocyanate, n-butyl isocyanate, phenyl isocyanate, 4-t-butyl isocyanate, acryloyloxyethyl isocyanate, methacryloyloxyethyl isocyanate, 4-methacryloyloxybutyl isocyanate, 4-acryloylphenyl isocyanate and 4-vinylphenyl isocyanate.

Suitable ethylenically unsaturated primary alcohols for use in the preparation of the compounds of the invention are the hydroxyalkyl acrylates having the formula:

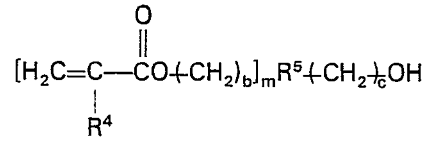

V

in which $R^4$, $R^5$, m and c are the same as defined for compounds of Formula I. Included among suitable hydroxyalkyl acrylates are the monoacrylate and monomethacrylate esters of aliphatic diols such as

ethyleneglycol, propyleneglycol, butyleneglycol, hexamethyleneglycol, diethyleneglycol, and dimethylolcyclohexane; the diacrylates and dimethacrylates of aliphatic triols such as trimethylolmethane, 1,1,1-trimethylolpropane, 1,2,3-trimethylolpropane; the triacrylates and trimethacrylates of aliphatic tetrols such as pentaerythritol, 1,1,2,2-tetramethylolethane and 1,1,3,3-tetramethylopropane; the tetraacrylates and tetramethacrylates of polyols such as dipentaerythritol and 1,1,1,2,2-pentamethylolethane; and the pentaacrylates and pentamethacrylates of polyols such as tripentaerythritol and hexamethylolethane.

Polymerization initiators suitable for use in the crosslinkable compositions of the invention are compounds which liberate or generate a free-radical on addition of energy. Such initiators include peroxy, azo, and redox systems each of which are well known and are described frequently in polymerization art, e.g. Chapter II of *Photochemistry*, by Calvert and Pitts, John Wiley & Sons (1966). Included among free-radical initiators are conventional heat activated catalysts such as organic peroxides and organic hydroperoxides; examples are benzoyl peroxide, tertiary-butyl perbenzoate, cumene hydroperoxide, azobis(isobutyronitrile) and the like. The preferred catalysts are photopolymerization initiators which facilitate polymerization when the composition is irradiated. Included among such initiators are acyloin and derivatives thereof, such as benzoin, benzoin methyl ether, benzoin ethyl ether, benzoin isopropyl ether, benzoin isobutyl ether, and $\alpha$-methylbenzoin; diketones such as benzil and diacetyl, etc; organic sulfides such as diphenyl monosulfide, diphenyl disulfide, decyl phenyl sulfide, and tetramethylthiuram monosulfide; S-acyl dithiocarbamates, such as S-benzoyl-N,N-dimethyldithiocarbamate; phenones such as acetophenone, $\alpha$, $\alpha$, $\alpha$,-tribromacetophenone; $\alpha$,$\alpha$-diethoxyacetophenone, 2,2-dimethoxy-2-phenylacetophenone, o-nitro-$\alpha$,$\alpha$,$\alpha$-tribromacetophenone benzophenone, and p,p'-tetramethyldiaminobenzophenone; aromatic iodonium and aromatic sulfonium salts; sulfonyl halides such as p-toluenesulfonyl chloride, 1-naphthalene-sulfonyl chloride, 2-naphthalenesulfonyl chloride, 1-3benzenedisulfonyl chloride, 2,4-dinitrobenzenesulfonyl bromide and p-acetamidobenzenesulfonyl chloride. Normally the initiator is used in amounts ranging from about 0.01 to 5% by weight of the total polymerizable composition. When the quantity is less than 0.01% by weight, the polymerization rate becomes extremely low. If the initiator is used in excess of 5% by weight, no correspondingly improved effect can be expected. Thus, addition of such greater quantity is economically unjustified. Preferably, about 0.25 to 1.0% of initiator is used in the polymerizable compositions.

The crosslinkable compositions of the invention are preferably diluted with an ethylenically unsaturated monomer. Suitable ethylenically unsaturated monomers include methyl methacrylate, ethyl acrylate, 2-ethylhexyl acrylate, cyclohexyl acrylate, styrene, 2-chlorostyrene, 2,4-dichlorostyrene, acrylic acid, acrylamide, acrylonitrile, t-butyl acrylate, methyl acrylate, butyl acrylate, 2-(N-butylcarbamyl)ether methacrylate and 2-(N-butylcarbamyl)ether methacrylate and 2-(N-ethylcarbamyl) ethyl methacrylate. Other diluting monomers that can be incorporated into the composition of the invention include 1,4-butylene dimethacrylate or acrylate, ethylene dimethacrylate, hexanediol diacrylate or dimethacrylate, glyceryl diacrylate or methacrylate, glyceryl triacrylate or trimethacrylate, pentaerythritol triacrylate or trimethacrylate, pentaerythritol tetraacrylate or tetramethacrylate, diallyl phthalate, dipentaerythritol pentaacryalte neopentylglycol triacrylate and 1,3,5-tri(2-methacrylate-oxyethyl)-*s*-triazine.

The compositions of the present invention may also contain compounds such as those disclosed in U.S. Patent No. 3,808,266:

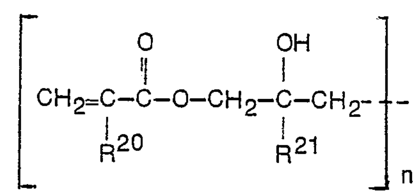 A

wherein

$R^{20}$ is hydrogen or methyl,

$R^{21}$ is hydrogen or methyl,

n is 2 or 3, and

A is as defined for the materials of U.S. Patent No. 3,821,098, and is

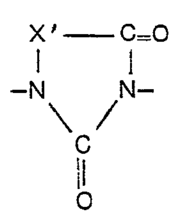

wherein X′ represents a divalent radical which is necessary for the completion of a five-or six-membered, unsubstituted or substituted, heterocyclic ring.

The radical X′ in the N-heterocyclic grouping of formula I can be, e.g., a radical of the formulae:

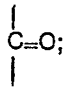 ; 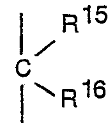 ; 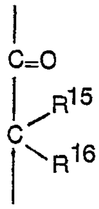 ; 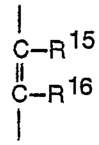 ; 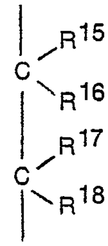 ; or 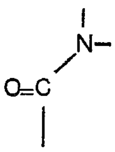

wherein $R^{15}$, $R^{16}$, $R^{17}$ and $R^{18}$ can each independently represent a hydrogen atom or an alkyl group, preferably a lower alkyl group having 1—4 carbon atoms, an alkenyl group, preferably a lower alkylene group having 1—4 carbon atoms, a cycloalkyl group (preferably of 3 to 8 carbon atoms), or an unsubstituted or substituted phenyl group. The valence of the nitrogen atom on the last group may be satisfied by hydrogen, aliphatic, or aromatic groups $R^{19}$, preferably of no more than eight carbon atoms. The aliphatic groups may be alkyl groups for example, and the cromatic group may be phenyl or alkylphenyl groups, for example.

The crosslinkable composition can also contain a viscosity modifier or binder. Generally, up to about 50 percent by weight of a compatible polymer is used. Preferably, the polymer is an acrylic polymer such as poly(acrylic acid), a poly(methacrylic acid), poly(methyl methacrylate), poly(vinyl chloride), poly(vinyl acetate, poly(vinyl butyral) and the like. Other polymers include polyethers, polyesters, polylactones, polyamides, polyurethanes, cellulose derivatives, polysiloxanes and the like.

The compositions of the invention can also include a variety of addenda utilized for their known purpose, such as stabilizers, inhibitors, lubricants, flexibilizers, pigments, carbon black, dyes, reinforcing fillers such as finely divided silica, non-reinforcing fillers such as diatomaceous earth, metal oxides, asbestos, fiberglass, glass bubbles, talc, etc. Fillers can generally be used in proportions up to about 200 percent by weight of the curable components but preferably are used up to about 50 percent by weight. Where the polymerizing energy is radiation, it is desirable that the addenda be transparent to the radiation.

The compositions of the invention are prepared by simply mixing (under "safe light" conditions if the composition is to be sensitized to visible light) the polymerization catalyst and sensitizer (where used), the poly(ethylenically unsaturated alkoxyalkyl)heterocyclic compound, diluting monomers, binders and addenda. Inert solvents may be employed if desired when effecting this mixture. Examples of suitable solvents are methanol, ethanol, acetone, acetonitrile and includes any solvent which does not react with the components of the mixture.

Utility

The crosslinkable compositions of the invention can be used as adhesives, caulking and sealing compositions, casting and molding compositions, potting and encapsulating compositions, impregnating and coating compositions, etc., depending of the particular combination of components. Where the polymerization catalyst is a photoinitiator, the composition can be a composition for *in situ* curing because of this insensitivity to oxygen.

The photopolymerizable compositions are particularly suitable for applications in the field of protective coatings and graphic arts because of their superior abrasion-resistance and adhesion to many rigid, resilient and flexible substrates such as metals, plastics, rubber, glass, paper wood, and ceramics; their excellent resistance to most solvents and chemicals; their excellent flexibility and weatherability; and their capability for forming high resolution images. Among such uses are water or water/alcohol developable resists for chemical milling, gravure images, offset plates, stencil making, screenless lithography, particulate binders as in microtaggants, relief printing plates, printed circuits, electron beam curing adhesives, radiation and protective coatings for glass, metal surfaces and the like and abrasion resistant coatings on a wide range of materials such as opthalmic lenses, light control films, and organic polymeric resin surfaces in general. Priming layers may be used if desired, and in some cases may be necessary.

The coatings of the present invention find application useful on substantially any solid substrate. Because the coatings of the present invention can be cured by radiation, even highly temperature sensitive substrates can be coated. The substrates may be in substantially any form, such as sheets, films, fibers, fabrics and shaped solid objects. Amongst the substrates particularly finding advantages with coatings of the present invention are polymeric resins, including both thermoplastic and thermoset resins (e.g., polyesters, polyethers, polyamides, polyurethanes, polycarbonates, polyacrylates, polyolefins, polyvinyls cellulosesters, epoxy resins, polysiloxanes, etc.), ceramic substrate, including glass, fused ceramic sheetings, and fibers, metals and metallized surfaces, natural cellulosic materials,

including wood and paper, natural resins, including rubber and gelatin and other various solid surfaces. The coatings are useful particularly on refractive substrates (e.g., lenses, prisms and the like) as well as reflective substrates (street signs, mirrors, etc). They are also useful on metallized polymeric film which is transparent and used as a light screen on windows.

Where the coating compositions of this invention are not naturally adherent to the particular substrate selected, primer compositions, comprising single ingredients or blends of materials, may be used to improve the bond of the coating to the substrate. Texturizing, chemical, or physical treatment of the surface may also be used to improve bonding. The coatings of the invention are generally between 0.5 and 500 microns thick, preferably between 1 and 50 microns, and most preferably between 3 and 25 microns.

Particularly useful substrates for application of the coatings of the present invention would be those requiring transparent protective coatings. Finished photographic prints and films, paintings, transparencies, car windshields, painted surfaces, instant film (i.e., film which does not require external application of developing chemistry), photothermographic and thermographic paper and film, photoconductive substrates, opthalmic lenses, liquid crystal displays, motion picture film, street and traffic signs, reflective surfaces, retroreflective surfaces, traffic lights, and many other substrates are usefully coated according to the practice of the present invention. These coatings are particularly useful on optically funtional surfaces or elements, particularly polarizing elements. These include both polymeric film type polarizers and the solvent-coated type polarizers such as are described in U.S. Paetnts 2,400,877; 2,481,830; and 2,544,659.

Where the polymerization initiator is a photoinitiator, the composition can be a composition for *in situ* curing because of its insensitivity to oxygen.

The photopolymerizable compositions are particularly suitable for applications in the field of protective coatings and graphic arts because of their superior abrasion-resistance and adhesion to many rigid, resilient and flexible substrates such as metals, metal oxides, plastics, rubber, glass, paper, wood, and ceramics; their excellent resistance to most solvents and chemicals; their excellent flexibility and weatherability; and their capability for forming high resolution images.

The photopolymerization of the compositions of the invention occurs on exposure of the compositions to any source of radiation emitting actinic radiation at a wavelength within the ultraviolet and visible spectral regions. Suitable sources of radiation include mercury, xenon, carbon arc and tungsten filament lamps, sunlight, etc. Exposures may be from less than about 1 second to 10 minutes or more depending upon the amounts of the particular polymerizable materials and photopolymerization catalyst being utilized and depending upon the radiation source, distance from the source, and the thickness of the coating to be cured. The compositions may also be polymerized by exposure to electron beam irradiation. Generally speaking, the dosage necessary is from less than 1 megarad to 100 megarad or more. One of the major advantages with using electron beam curing is that highly pigmented compositions can be effectively cured at a faster rate than by mere exposure to actinic radiation.

It has been found that at least 15% by weight of the polymer layer should comprise the compounds of the present invention in order to obtain abrasion resistance.

These and other features of the present invention will be shown with reference to the following Examples.

Examples 1

*Preparation of 1,3-Bis(3-[2,2,2-(triacryloyloxymethyl) ethoxy-2-hydroxypropyl)-5,5-dimethyl-2,4-imidazolidinedione*

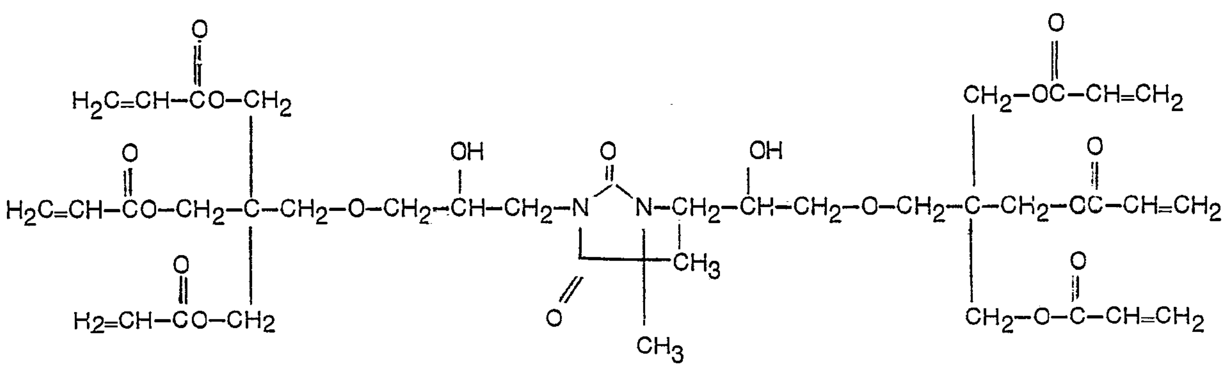

Compound A

Pentaerythritol triacrylate (44.3 g, 0.1 moles, hydroxyl equivalent weight of 443), .025 g 4-methoxy-phenol, and 0.4 g borontrifluoride etherate were charged into a 250 ml three-necked round bottom flask equipped with mechanical stirrer, pressure equalizing dropping funnel, reflux condenser,

and a $CaSO_4$ drying tube. (It is to be noted that most commercially available pentaerythritol triacrylate is contaminated with acrylated impurities.) The reaction flask was heated to 60°C and 13.8 g of 1,3-bis(2,3-epoxypropyl)-5,5-dimethyl-2,4-imidazolidinedione (0.1 m epoxide equivalency) in 5 ml chloroform was added dropwise over 45 minutes. After the addition, the reaction flask temperature was raised to 85°C and stirred for 11.5 hours. After this time, titration of an aliquote for unreacted epoxide indicated that the reaction was greater than 99% complete. The chloroform was removed by vacuum distillation leaving as residue a viscous liquid that contains predominantly compounds of the structure of Compound A. Photocurable impurities introduced with the pentaerythritol triacrylate can be removed by trituration with diethyl ether.

A mixture of the liquid and 2% by weight of the photopolymerization initiator 2,2-dimethoxy-2-phenyl-acetophenone was coated onto 12 m polyester film and dried to provide a 2.5 $\mu$m layer. The layer was then cured in a UV Processor, Model No. CC 1202 N/A (manufactured by Radiation Polymer Co.) after one pass at 12 m/min. (40 feet/min.) under an 80 watts/cm (220 watts/inch) medium pressure mercury lamp. The cured layer exhibited 95—100% cross-hatch adhesion, 2—7% Taber Haze, 13—16% haze in the Gardner Falling Sand Abrader (i.e., tested according to ASTM Designation D1003—64 (Procedure A)) and excellent resistance to abrasion by steel wool. The layer was unaffected by treatment with ethanol, acetone, ethyl acetate, toluene, hexane, aqueous sodium hydroxide and 10% aqueous hydrochloric acid.

Examples 2—3

*Preparation of 1,3-Bis[3-(2-acryloyloxyethoxy)-2-hydroxypropyl]-5,5-dimethyl-2,4-imidazolidinedione*

Compound B

Distilled hydroxyethyl acrylate (46.4 g, 0.4 moles), 0.065 g 4-methoxyphenol, and 1.0 g borontrifluoride etherate were charged into a 250 ml three-necked round bottom flask equipped with mechanical stirrer, pressure equalizing dropping funnel, reflux condenser, and $CaSO_4$ drying tube. The reaction flask was heated to 60°C and 55.2 g 1,3-bis(2,3-epoxypropyl)-5,5-dimethyl-2,4-imidazolidenedione in 10 ml chloroform was added dropwise over 30 minutes. The reaction flask temperature was raised to 75°C for 11 hours. At this time titration of residual epoxide groups indicated that the reaction was 97% complete. The volatiles were removed by vacuum distillation leaving as residue a liquid.

A layer of the compound containing 2% of 2,2-dimethoxy-2-phenylacetophenone was prepared and cured as in Example 1. The cured layer had chemical resistance similar to that of the layer of Example 1.

The analogous dimethacryloyl derivative (Compound C) was prepared in a similar manner utilizing 2-hydroxyethyl methacrylate in place of 2-hydroxyethyl acrylate. Layers prepared and cured with Compound C in the same manner as with Compound B had characteristics similar to those layers formed from Compound B.

Example 4

Preparation of 1-[3-(2-acryloyloxyethoxy)-2-hydroxypropyl]
-3[3-(2-acryloyloxyethoxy)-2-[[3-carboxyacryloyloxy]]propyl]
-5,5-dimethyl-2,4-imidazolidinedione

Compound D

Compound B (10.0 g, 0.025 moles from Example 2) and 2.4 g maleic anhydride were charged into a 100 ml three-necked round bottom flask equipped with mechanical stirrer, reflux condenser, and $CaSO_4$ drying tube. The reaction was heated at 80°C for six hours. At this time the reaction was terminated to yield a viscous slightly yellow liquid displaying a strong, broad infrared spectral absorbance centered at 3000 $cm^{-1}$, characteristic for carboxylic acids.

A layer of this material containing 2% of 2,2-dimethoxy-phenylacetophenone was prepared as in Example 1. This layer was cured to insolubility with a Hanovia 3D960 mercury arc lamp in 60 seconds. The sample was 6 cm from the light source.

Examples 5—10

Various amounts of Compounds A and B were mixed with trimethylolpropanetriacrylate (TMPTA) and 2% by weight of the photopolymerization initiator of Example 1 added. Each mixture was diluted with an equal weight of acetone and coated onto 12 $\mu$m polyester film and dried. The dried coating was 2.5 $\mu$m thick. On exposure in air at a distance of 6 cm the radiation from a 100 watt Hanovia 3D690 lamp and the time measured at which each become insoluble in acetone. The data obtained is recorded in Table 1.

TABLE I

| Exp. No. | Composition Compound (%) | TMPTA | Cure Time (Sec.) |
|---|---|---|---|
| 5 | None | 100 | 600 |
| 6 | A (17) | 83 | 80 |
| 7 | A (28) | 72 | 60 |
| 8 | A (50) | 50 | 50 |
| 9 | A (100) | 0 | 10 |
| 10 | B (100) | 0 | 30 |

By reference to Table I it can be seen that TMPTA requires 10 minutes to reach insolubility and that with the addition of 17% of Compound A (from Example 1) the cure time is reduced to 80 seconds and with increasing amounts of A, the composition cures faster until at 100% A, the composition under the stated conditions cures in only 10 seconds. Comparable results can be obtained with Compound B.

Example 11

A layer, 2.5 μm in thickness, of Compound B containing 2% of the photopolymerization catalyst of Example 1 on 12 μm polyester film was prepared as described in Example 1. A patterned template was placed over the layer and exposed in the UV processor to one pass at 12 m/min. of an 80 watts/cm lamp. The exposed sheet was washed with cold water leaving an image having excellent resolution.

Example 12

One part polyacrylic acid, one part compound A from Example 1, five parts water, five parts ethanol and 0.02 parts of the photopolymerization catalyst of Example 1 were mixed together to form a solution. A layer 5.0 μm in thickness of this solution was coated onto 12 μm polyester as described in Example 1. A patterned template was placed over the layer as exposed by a Hanovia 3D690 mercury arc lamp at a distance of 6 cm for two minutes. The exposed sheet was developed with cold water leaving an image having excellent resolution.

Example 13

*Preparation of Compound E*

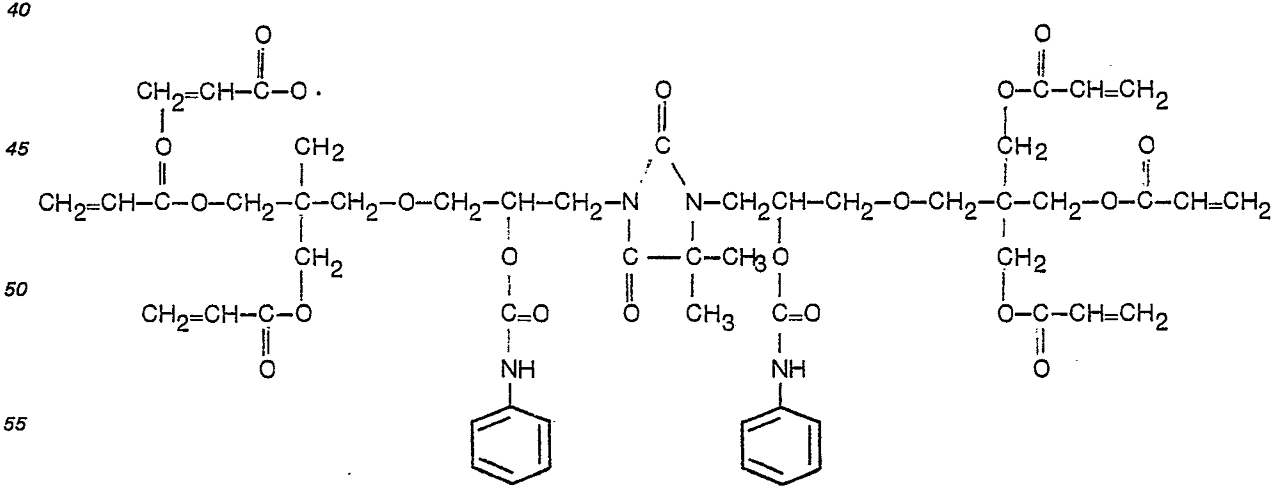

Compound A (20.4 g from Example 1) and 10.6 ml dry tetrahydrofuran were dissolved in a 250 ml 3-necked round bottom flask equipped with a magnetic stirrer, reflux condenser, pressure equalizing dropping funnel and $CaSO_4$ drying tube. 5.7 g phenylisocyanate was added dropwise over the course of five minutes. The reaction was terminated after stirring for twenty hours at room temperature. The lack of an isocyanate infrared absorption band indicates the reaction of the isocyanate to be quantitative.

A layer of this material containing 2% of the photopolymerization catalyst of Example 1 was prepared as in Example 1. This layer was cured to insolubility with a Hanovia 3D690 mercury arc lamp in 15 seconds. The sample was 6 cm from the light source.

Example 14 (Comparative)

*Preparation of 1,3-Bis[3-(2-allyloxyethoxy)-2-hydroxypropyl]-5,5-dimethyl-2,4-imidazolidine-dione*

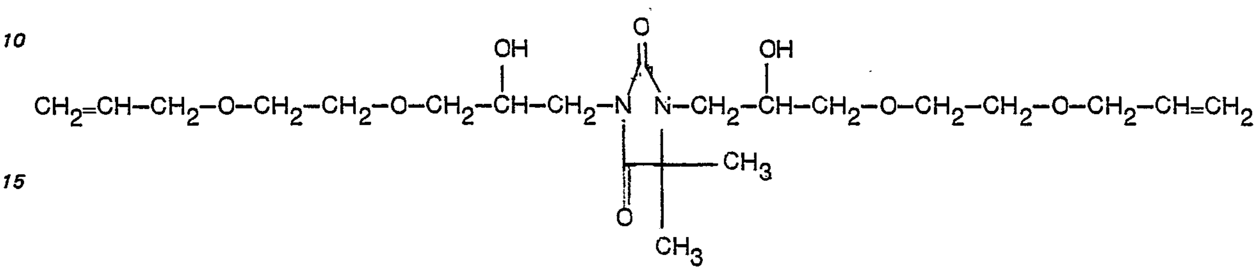

Compound F

2-allyloxyethanol (20.43 g, 0.1 moles), 0.03 g 4-methoxyphenol, and 0.30 g borontrifluoride etherate were charged into a 250 ml three-necked round bottom flask equipped with mechanical stirrer, pressure equalizing dropping funnel, reflux condenser and $CaSO_4$ drying tube. The reaction flask temperature was heated to 80°C and 13.8 g 1,3-bis(2,3-epoxypropyl)-5,5-dimethyl-2,4-imidazolidine-dione in 4.5 g chloroform was added dropwise over 30 minutes. The reaction was maintained at 80° for 17 hours. At this time titration of residual epoxide groups indicated that the reaction was 99% complete. The chloroform was removed by vacuum distillation leaving as residue a colorless liquid.

Example 15

Into a 250 ml three-necked round bottom flask equipped with mechanical stirrer, pressure equalizing dropping funnel, reflux condenser, and calcium sulfate drying tube were charged 103.0 g pentaerythritol triacrylate (hydroxy equivalent weight of 515), 23.2 g 2-hydroxyethyl acrylate (0.2 moles), 0.08 g 4-methoxyphenol, and 1.0 g borontrifluoride etherate. The reaction flask was heated to 75°C and 55.2 g (0.40 molar epoxy equivalency) 1,3-bis(2,3-epoxypropyl)-5,5-dimethyl-2,4-imidazolidinedione in 20 ml chloroform was added dropwise over one hour. After the addition, the reaction flask temperature was raised to 88°C and stirred for 18.0 hours. At this time, titration of an aliquot for unreacted epoxide indicated the reaction was treater than 99% complete. The volatiles were removed by vacuum distillation leaving a viscous liquid which contains a mixture of bis(tri-acyloyl)-, bis(monoacryloyl)-, and the unsymmetrical monoacryloyl-triacryloyl-imidazolidinedione, and impurities, introduced with the pentaerythritol triacrylate.

A layer of the reaction product of Example 15, prepared to contain 2% Irgacure 651 and cured as described in Example 1, had abrasion and chemical resistance characteristics similar to those of the layer of Example 1.

Example 16

Mixtures of compounds A—F and 2% by weight of an appropriate photoinitiator were coated onto 12 μm polyester and dried to provide a 2.5 μm layer. For compounds A through E, 2,2-dimethoxy-2-phenyl-acetophenone was used as a photoinitiator; for compound F benzophenone was used as a photoinitiator, as specified in USP 3925349. The layers were then cured in a UV Processor, Model No. CC 1202 N/A (manufactured by Radiation Polymer Co.). An 80 watt/cm (200 watts/inch) medium pressure mercury arc lamp was used as the radiation source. A film was considered cured when it displayed mar resistance after treatment with ethanol. All films were cured in one pass at a given table speed unless indicated otherwise.

The cure rates in air for compounds A—C of this invention are slower only by a factor of from 0.45—0.90 than the corresponding cure rates under a nitrogen atmosphere. This is in marked contrast to the cure rates in air of acrylates D and E which are slower than their corresponding cure rates under a nitrogen atmosphere by at least a factor of 0.006.

Compound F is described in USP 3925349 as photocurable in the presence of air. A film of this material cures at similar rates in the presence or absence of oxygen, but fails to display good mar resistance.

Example 17

A solution containing 2.3 g of compound A from Example 16, 2.3 g acetone, and 0.033 g diethoxyacetophenone was coated onto a 12 μm polyethylene sheet with a #14 Meyer bar. The layer was dried and placed in a mounting for a Perkin Elmer No. 257 infrared spectrophotometer. The absor-

bances at 1630 $cm^{-1}$ and 810 $cm^{-1}$, characteristic of acrylate unsaturation, were recorded. The mounting was removed from the spectrophotometer and passed through a UV processor as described in Example 16 at 50 ft/min. The mounting was returned to the spectrophotometer and the absorbances at 1630 $cm^{-1}$ and 810 $^{-1}$ were recorded and compared to the initial absorbances. This experiment showed that 70—80% of the acrylate functionality was consumed under the curing conditions. Five additional passes of this film at 50 ft/min in the UV processor failed to show any significant decrease in the acrylate infrared absorbances.

The same experiment was repeated, except that the film was cured under a nitrogen atmosphere. Once again 70—80% of the acrylate functionality was consumed in one pass at 50 ft/min with no additional crosslinking being observed with additional curing time.

The experiments described above indicate that under these curing conditions there is no observable difference in the degree of crosslinking for Compound A, Example 1, in the presence or absence of atmospheric oxygen.

Example 18

One part polyacrylic acid, one part compound A from Example 1, five parts water, five parts ethanol and 0.02 parts of 2,2-dimethoxy-2-phenyl-acetophenone were mixed together to form a solution. A layer 5.0 μm in thickness of this solution was coated onto 12 μm polyester as described in Example 1. A patterned template was placed over the layer and exposed by a Hanovia 3D690 mercury arc lamp in air at a distance of 6 cm for two minutes. The exposed sheet was developed with cold water leaving an image having excellent resolution.

Examples 19—26

Coatings about 5 μm thick were prepared by coating using a #20 Meyer bar onto about 12 μm polyethylene film primed with polyvinylidene chloride 50% solutions in ethyl acetate (other solvents such as ketones and lower alcohols are equally suitable) and drying mixtures of Compound B of Example 2, zero to 100% of Compound 2 of Example 1, and pentaerythritol triacrylate (PTA) based on total weight of Compounds A, B and PTA. To each solution had been added, as photoinitiator, 1% of Irgacure 651 (similar results were obtained with, -diethoxyacetophenone and benzoin ethylether) and, as coating aid, 0.01% of a fluorocarbon or silicone surfactant. The dried coatings were then cured by one pass in a Model 1202 AN (PPG) Ultraviolet Processor (manufactured by Radiation Polymer Company) operated at about 12 m/min with an 80 watt/cm medium pressure mercury vapour lamp 15 cm from the surface of the layer without exclusion of air. The cured layer was tested for cross-hatch adhesion, Taber Wheel abrasion, resistance to steel wool, and falling sand abrasion. The results are recorded in Table 2.

TABLE 2

| Exp. No. | %1 | %PTA | %2 | Cross-Hatch Adhesion % | Taber % Haze | Abrasion Resistance Falling sand % Haze | Steel Wool |
|---|---|---|---|---|---|---|---|
| 19 | 75 | 25 | 0 | 100 | 2—7 | 13—16 | Excellent |
| 20 | 67.5 | 22.5 | 10 | 100 | 2—7 | 13—16 | " |
| 21 | 60 | 20 | 20 | 100 | 2—7 | 13—16 | " |
| 22 | 52.5 | 17.5 | 30 | 100 | 5—9 | | " |
| 23 | 45 | 15 | 40 | 100 | 5—9 | | " |
| 24 | 37.5 | 12.5 | 50 | 100 | 5—9 | | " |
| 25 | 30 | 10 | 60 | 100 | 28—34 | | Fair |
| 26 | 0 | 0 | 100 | | | | |

Table 2 shows that coatings prepared from the hexaacryloyleoxyhydantoin, Compound A, and up to more than 50% of the diacryloyloxyhydantoin, Compound B, have excellent resistance to abrasion as measured by Taber, falling sand and steel wool procedures. At up to about 30% Compound B, abrasion resistance is superior. When coatings were prepared as for Examples 3—10 but using photoinitiator concentrations from 0.4 to 3% and tested, similar abrasion resistance and cross-hatch adhesion was obtained.

**Claims**

1. Compounds of the general formula:

$$A^1—Z—A^2$$

in which $A^1$ and $A^2$ are independently alkoxyalkyl groups having terminal ethylenic unsaturation and having the general formula:

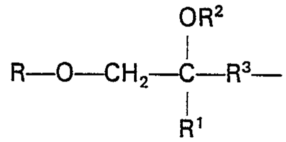

in which R—O— is a monovalent residue of an aliphatic terminally unsaturated primary alcohol, ROH, in which R has the formula:

$$[E\text{-}(CH_2)_b\text{-}]_m R^5\text{-}(CH_2)\text{-}_c$$

in which:

E is 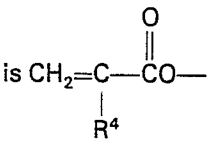

b is zero or an integer of from 1 to 6,
c is an integer of from 1 to 6,
$R^1$ and $R^4$ are independently hydrogen or methyl,
$R^5$ is an aliphatic group having 1 to 15 carbon atoms which may be interrupted with up to two groups selected from ether oxygen groups and

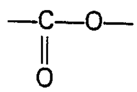

and has a valence of m + 1 in which m is an integer of 1 to 5,
$R^2$ is selected from hydrogen,

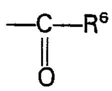 and 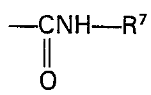

in which
$R^6$ is an alkyl or alkenyl group either of which groups may be substituted with a phenyl or carboxyl group,
$R^7$ is an aliphatic or aromatic group,
$R^3$ is an alkylene group having 1 to 6 carbon atoms and up to one —O— radical in the group, and
Z is a heterocyclic group of the formula:

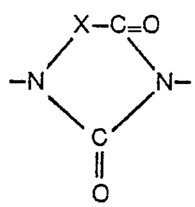

in which X is a divalent group required to complete a 5- or 6-membered heterocyclic ring and is selected from

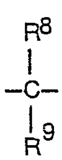 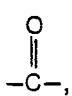, 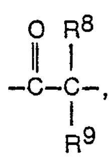, 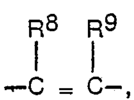, 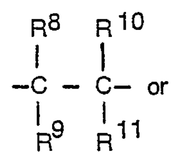 or 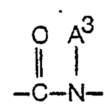

in which $R^8$, $R^9$, $R^{10}$ and $R^{11}$ are independently selected from hydrogen, alkyl groups of 1 to 12 carbon atoms, cycloalkyl groups of 3 to 6 carbon atoms, and optionally substituted phenyl groups of 6 to 12 carbon atoms, and

$A^3$ is as defined above for $A^1$ and $A^2$.

2. A compound as claimed in Claim 1, in which $R^2$ is

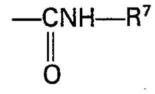

in which $R^7$ is an alkyl group, phenyl group, acryloyloxyalkyl group or methacryloxyalkyl group.

3. A compound as claimed in Claim 1, which $R^2$ is hydrogen.

4. A compound as claimed in Claim 1, in which $R^2$ is

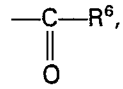

in which $R^6$ is an alkenyl or alkyl group of 2 to 5 carbon atoms.

5. A compound as claimed in any preceding claim, in which X is

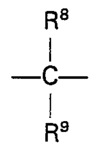

6. A compound as claimed in Claim 5, in which X is

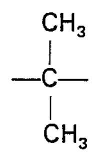

7. A compound as claimed in any preceding claim, in which m is 2 to 5.

8. A coated substrate comprising a substrate having adhered on at least one surface thereof a crosslinked polymeric coating derived from a polymerizable composition comprising at least 15% by weight of a compound as claimed in any preceding claim.

9. A coated substrate as claimed in Claim 8, in which the substrate is an organic polymeric resin.

10. A coated substrate as claimed in Claim 8, in which the substrate is a refractive or reflective surface.

## Patentansprüche

1. Verbindungen der Formel

$$A^1—Z—A^2$$

in der $A^1$ und $A^2$ unabhängig voneinander Alkoxyalkylrest mit endständiger äthylenischer Unsättigung sind und die allgemeine Formel aufweisen:

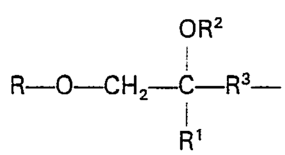

in der R—O— ein einwertiger Rest eines Aliphatischen, endständig ungesättigten primären Alkohols, ROH, ist, in dem R die Formel hat:

$$[E{+}CH_2{+}_b]_m R^5{+}CH_2{+}_c$$

in der:

E

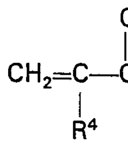 ist,

b null oder eine ganze Zahl im Wert von 1 bis 6 ist,

c eine ganze Zahl im Wert von 1 bis 6 ist,

$R^1$ und $R^4$ unabhängig voneinander Wasserstoff- oder Methylgruppen sind,

$R^5$ ein aliphatischer Rest mit 1 bis 15 Kohlenstoffatomen ist, der durch bis zu zwei Äther-Sauerstoffatome oder Reste der Formel

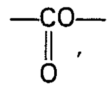 ,

unterbrochen sein kann und eine Wertigkeit von m + 1 aufweist, wobei m eine ganze Zahl von 1 bis 5 darstellt,

$R^2$ Wasserstoff oder eine Rest der Formel

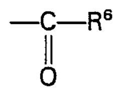 oder 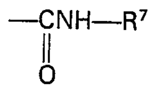

ist, in der

$R^6$ ein Alkyl- oder Alkenylrest ist, die beide mit einer Phenyl oder Carboxylgruppe substituiert sein können,

$R^7$ ein aliphatischer oder aromatischer Rest ist,

$R^3$ einen Alkylenrest mit 1 bis 6 Kohlenstoffatomen und bis zu einer Gruppe der Formel —O— im Rest darstellt, und

Z ein heterocyclischer Rest der Formel

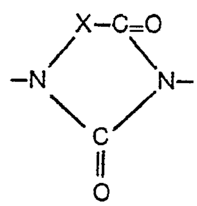

ist,

in der X eine zweiwertige Gruppe ist, die zur Vervollständigung eines 5- oder 6-gliedrigen heterocyclischen Rings erforderlich ist, und ausgewählt ist aus

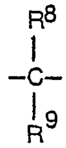 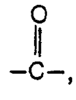, 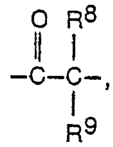, 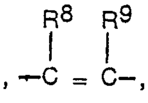, 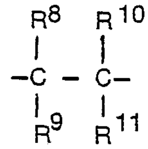 oder 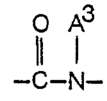

in denen, $R^8$, $R^9$, $R^{10}$ und $R^{11}$ unabhängig voneinander Wasserstoff, Alkylreste mit 1 bis 12 Kohlenstoffatomen, Cycloalkylreste mit 3 bis 6 Kohlenstoffatomen und gegebenenfalls substituierte Phenylgruppen mit 6 bis 12 Kohlenstoffatomen bedeuten und

$A^3$ wie vorstehend $A^1$ und $A^2$ definiert ist.

2. Eine Verbindung nach Anspruch 1, in der $R^2$ der Rest

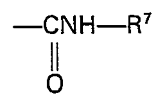

ist, in dem $R^7$ ein Alkyl-, Phenyl-, Acryloyloxyalkyl- oder Methacryloyloxyalkyl rest ist.

3. Eine Verbindung nach Anspruch 1, in der $R^2$ Wasserstoff ist.

4. Eine Verbindung nach Anspruch 1, in der $R^2$ der Rest

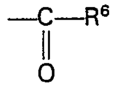

ist, in dem $R^6$ ein Alkenyl- oder Alkyl rest mit 2 bis 5 Kohlenstoffatomen ist.

5. Eine Verbindung nach einem der vorangehenden Ansprüche, in der X der Rest

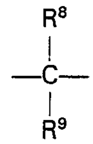

ist.

6. Eine Verbindung nach Anspruch 5, in der X

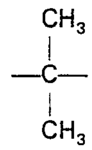

ist.

7. Eine Verbindung nach einem der vorangehenden Ansprüche, in der m 2 bis 5 ist.

8. Ein beschichtetes Substrat, umfassend ein Substrat, das auf mindestens einer seiner Oberflächen eine vernetzte polymere Beschichtung angebracht hat, die von einer polymerisierbaren Zusammensetzung abgeleitet ist, welche mindestens 15 Gewichtsprozent einer Verbindung nach einem der vorangehenden Ansprüche umfaßt.

9. Ein beschichtetes Substrat nach Anspruch 8, wobei das Substrat ein organisches polymeres Harz ist.

10. Ein neschichtetes Substrat nach Anspruch 8, wobei das Substrat eine lichtbrechende oder reflektierende Oberfläche aufweist.

**Revendications**

1. Composés de formule:

$$A^1—Z—A^2$$

où $A^1$ et $A^2$ sont indépendamment des groupes alcoxyalkyles ayant une insaturation éthylénique terminale et ayant pour formule générale:

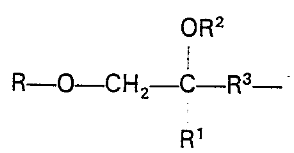

ou R—O— est un reste monovalent d'un alcool primaire aliphatique à insaturation terminale, ROH, où a pour formule:

$$[E(CH_2)_b]_m R^5 (CH_2)_c$$

où:

E est 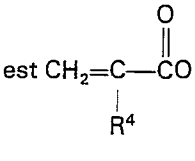

b est zéro ou un entier de 1 à 6,

c est un entier de 1 à 6

$R^1$ et $R^4$ sont indépendamment un hydrogène ou un méthyle,

$R^5$ est un groupe aliphatique ayant a à 15 atomes de carbone qui peut être interrompu avec jusqu'à deux groupes choisis parmi les groupes oxygènes de type éther et

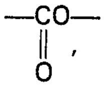,

et a une valence de m + 1 où m est un entier de 1 à 5,

$R^2$ est choisi parmi l'hydrogène,

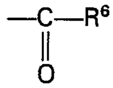 et 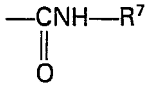

où

$R^6$ est un groupe alkyle ou alcényle, l'un ou l'autre de ces groupes pouvant être substitué par un groupe phényle ou carboxy,

$R^7$ est un groupe aliphatique ou aromatique,

$R^3$ est un groupe alkylène ayant 1 à 6 atomes de carbone et jusqu'à un radical —O— dans le groupe, et

Z est une groupe hétérocyclique de formule:

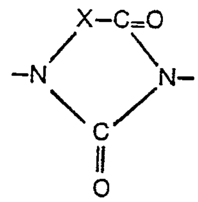

où X est un groupe divalente nécessaire pour réaliser un noyau hétérocyclique phentagonal ou hexagonal et est choisi parmi

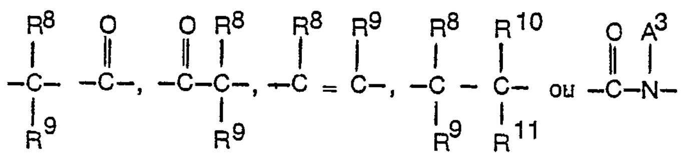

où $R^8$, $R^9$, $R^{10}$ et $R^{11}$ sont choisis indépendamment parmi l'hydrogène, les groupes alkyles de 1` 12 atomes de carbone, les groupes cycloalkyles de 3 à 6 atomes de carbone et les groupes phényles éventuellement substitués de 6 à 12 atomes de carbone, et

$A^3$ est comme défini ci-dessus pour $A^1$ et $A^2$.

2. Un composé comme revendiqué dans la revendication 1, où $R^2$ est

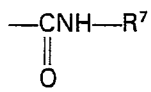

où $R^7$ est un groupe alkyle, un groupe phényle, un groupe acryloyloxyalkyle ou un groupe méthacryloyloxyalkyle.

3. Un composé comme revendiqué dans la revendication 1, où $R^2$ est un hydrogène.

4. Un composé comme revendiqué dans la revendication 1, où $R^2$ est

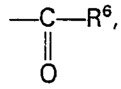

où $R^6$ est un groupe alcényle ou alkyle de 2 à 5 atomes de carbone.

5. Un composé comme revendiqué dans l'une quelconque des revendications précédentes, où X est

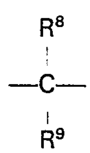

6. Un composé comme revendiqué dans la revendication 5, où X est

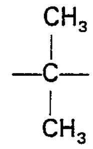

7. Un composé comme revendiqué dans l'une quelconque des revendications précédentes, oû m est 2 à 5.

8. Un substrat revêtu comprenant un substrat à au moins une des surfaces duquel adhère un revêtement polymère réticulé dérivant d'une composition polymérisable comprenant au moins 15% en poids d'un composé comme revendiqué dans l'une quelconque des revendications précédentes.

9. Un substrat revêtu comme revendiqué dans la revendication 8, dans lequel le substrat est une résine polymère organique.

10. Un substrat revêtu comme revendiqué dans la revendication 8, dans lequel le substrat est une surface réfringente or réfléchissante.